# EUROPÄISCHE PATENTANMELDUNG

(11) **EP 0 786 216 A2**
(43) Veröffentlichungstag der Anmeldung: **30.07.1997**
(21) Anmeldenummer: 97100390.0
(22) Anmeldetag: 11.01.1997
(51) Int. Cl.: A46B 3/18

(54) **Pflegestäbchen**

(30) Priorität: 26.01.1996 DE 29601191 U; 17.07.1996 DE 19628795; 19.10.1996 DE 19643241
(71) Anmelder: Wiegner, Georg, Tsimshatsui, Kowloon (HK)
(72) Erfinder: Wiegner, Georg, Tsimshatsui, Kowloon (CN); Kim, Hyeong Sook (Morin), Tsimshatsui, Kowloon (CN)
(74) Vertreter: Sparing - Röhl - Henseler Patentanwälte

(57) **Zusammenfassung**

Die Erfindung betrifft ein Pflegestäbchen mit einem länglichen Griffkörper, an dessen beiden freien Enden jeweils ein Pflegekörper angeordnet ist. Um das Pflegestäbchen universeller zu gestalten, ist vorgesehen, daß mindestens ein Pflegekörper gebildet ist von einem Bürstenkopf mit entlang eines Drahtes befestigten Borsten, und ein überstehendes freies Ende des Drahtes von einem tropfenförmigen Polymerüberzug umschlossen ist.

## Beschreibung

Die Erfindung betrifft ein Pflegestäbchen mit einem länglichen Griffkörper, an dessen beiden freien Enden jeweils ein Pflegekörper angeordnet ist.

Derartige Pflegestäbchen mit an beiden Enden befestigten Pflegekörpern, insbesondere Wattepolstern, werden für die tägliche Schönheitspflege, insbesondere Augenkosmetik, Maniküre, Lippenstiftkorrektur, vielfach eingesetzt.

Aufgabe der vorliegenden Erfindung ist es, das Einsatzgebiet der bekannten Pflegestäbchen universeller zu gestalten.

Diese Aufgabe wird dadurch gelöst, daß das Pflegestäbchen mindestens einen Bürstenkopf aufweist und dadurch das Einsatzgebiet vergrößert wird. Ferner können die beiden Pflegekörper unterschiedlich gestaltet sein, wodurch die Benutzungshäufigkeit beider Seiten eines Pflegestäbchens verbessert wird. Die Befestigung der Borsten an einem Draht erlaubt eine schlanke Gestaltung des Bürstenkopfes, so daß dieser als Interdentalbürstchen für die Zahnpflege einsetzbar ist. Eine Verletzungsgefahr durch das freie Drahtende wird durch dessen Einkapselung in einen tropfenförmigen Polymerüberzug vermieden. Mögliche scharfe Kanten werden eingebettet, wobei hierfür insbesondere gieß- und/oder schrumpffähige Polymerkunststoffe geeignet sind. Die tropfenförmige Ausbildung des Überzuges kann bezüglich der Dimensionierung derart gewählt werden, daß ein Einsatz als Interdentalbürstchen nicht behindert wird.

Durch die Anbringung des Bürstenkopfes entfällt gegenüber bekannten Pflegestäbchen zwar eines von zwei Wattepolstern, was jedoch keinen wesentlichen Nachteil mit sich bringt. Pflegestäbchen werden im allgemeinen nach einer Einmalverwendung bereits vernichtet, so daß der Vorrat in Form eines zweiten Wattepolsters im allgemeinen ungenutzt bleibt.

Die Befestigung des Bürstenkopfes an dem Griffkörper kann besonders zugfest durch Einkleben eines Teilstücks desselben in einen als extrudierte Hülse ausgebildeten Griffkörper vorgesehen sein.

Die Bürstenkopfart kann individuell gewählt werden, wobei die üblichen Bürstenarten, wie insbesondere Rundbürste und Flachbürste, einsetzbar sind. Weiterhin kann die Bürstenkopfform frei gewählt werden. Für Zahnreinigungszwecke bevorzugt sind konisch gestaltete Bürstenköpfe.

Als Material für den Bürstenkopf sind Naturborsten ebenso wie Kunststoffborsten verwendbar.

Der Griffkörper des Pflegestäbchens besteht vorzugsweise aus einem elastischen Kunststoffmaterial, wodurch die Führigkeit des Pflegestäbchens verbessert wird. Als Kunststoff verwendbar ist ein Celluloseester, insbesondere Cellulosepropionat oder Celluloseacetopropionat. Solche Celluloseester verleihen dem Griffkörper eine Elastizität, die verhindert, daß die mit der Hand geführten Pflegestäbchen eine zu hohe Kraftübertragung bewirken. Die Verletzungsgefahr ist dadurch wesentlich reduziert.

Die Pflegekörper eines Pflegestäbchens können beide als Bürstenkopf ausgebildet oder der Bürstenkopf mit einem anderen Pflegekörper, insbesondere einem Wattepolster, einem Zahnstocher oder einem Zahnseidenhalter kombiniert sein.

Weitere Ausgestaltungen und Vorteile der Erfindung sind der nachfolgenden Beschreibung und den Unteransprüchen zu entnehmen.

Die Erfindung wird nachstehend anhand der in der beigefügten Abbildung dargestellten Ausführungsbeispiele näher erläutert.
- Figur 1: zeigt schematisch eine Seitenansicht eines ersten Ausführungsbeispiels eines Pflegestäbchens,
- Figur 2: zeigt schematisch eine Seitenansicht eines zweiten Ausführungsbeispiels eines Pflegestäbchens,
- Figur 3: zeigt schematisch eine Seitenansicht eines dritten Ausführungsbeispiels eines Pflegestäbchens.
- Figur 4: zeigt schematisch eine Seitenansicht eines vierten Ausführungsbeispiels eines Pflegestäbchens.

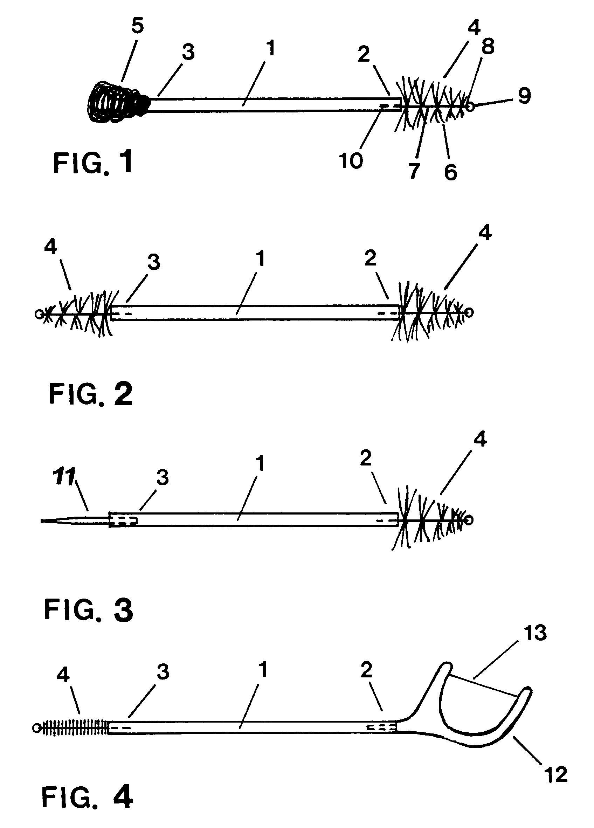

Das in Figur 1 dargestellte erste Ausführungsbeispiel eines Pflegestäbchens umfaßt einen länglichen Griffkörper 1, an dessen beiden freien Enden 2, 3 ein erster und zweiter Pflegekörper angeordnet sind. Als erster Pflegekörper ist an dem ersten freien Ende 2 ein Bürstenkopf 4 befestigt. Der zweite Pflegekörper an dem zweiten Ende 3 ist beliebig wählbar und ist hier als Wattepolster 5 ausgebildet. Die Abmessungen des Griffkörpers 1 sowie des Wattepolsters 5 als auch des Bürstenkopfes 4 sind auf einen Handgebrauch abgestellt und können an die Abmessungen bekannter Pflegestäbchen angepaßt sein.

Das Wattepolster 5 und der Bürstenkopf 4 erstrecken sich in Längsrichtung des Griffkörpers 1, wobei das Wattepolster 5 das zweite Ende 3 des Griffkörpers 1 vorzugsweise teilweise umwickelt, um eine formstabile Ausbildung zu besitzen.

Der Bürstenkopf 4 ist an dem ersten freien Ende 2 des Griffkörpers 1 befestigt und verlängert den Griffkörper 1 in Längsrichtung. Der Bürstenkopf 4 ist mit einer Vielzahl von Borsten 6 besetzt, die an einem Bürstenkörper befestigt sind. Der Bürstenkörper ist ein Draht 7, an dem die Borsten 6 festgeklemmt sind. Die Borsten 6 können nach einem wählbaren Muster angeordnet sein. Vorzugsweise ist der Bürstenkopf 4 als Rundbürste oder Flachbürste ausgebildet, wobei der Bürstenkopf 4 eine zylinderförmige, konische oder unstetig geformte Längserstreckung aufweisen kann. Der Draht 7 erstreckt sich mit einem zumindest geringfügigen Längenabschnitt über einen mit Borsten 6 bestückten Drahtabschnitt hinaus und bildet dort ein freies Ende 8, das von einem tropfenförmigen Polymerüberzug 9 ummantelt ist. Der Draht 7 ist also bewußt verlängert, insbesondere 1 bis 2,5 mm, und das daran befindliche freie Ende 8 ist von einem Kunststoffmaterial umschlossen, das tropfenförmig dieses Ende 8 umgibt und damit abrundet. Ein reines Abkappen des Drahtes 7 an seinem freien Ende 8, egal wie kurz, birgt wegen eines immer irgendwo freiliegenden Drahtendes eine Verletzungsgefahr, die hier ausgeschaltet wird. Scharfe Schnittkanten werden nämlich in das tropfenförmige Gebilde eingebettet. Selbst wenn der notwendigerweise sehr dünne Draht an seinem überstehenden Ende als geschlossene Schlaufe umgeformt ist, bildet eine solche Drahtspitze auch ungekappt eine erhebliche Verletzungsgefahr.

Als tropfenförmiger Polymerüberzug 9 bevorzugt ist ein gehärteter Duromertropfen, wobei als Duromer ein UP-Harz, Vinylesterharz oder Epoxidharz eingesetzt werden kann. Alternativ kann der tropfenförmige Polymerüberzug aus einem Thermoplast gearbeitet sein. Bevorzugt ist ferner, daß das für den Polymerüberzug verwendete Kunststoffmaterial schrumpffähig ist.

Als Material für den Bürstenkopf 4 sind Natur- und Kunststoffmaterialien verwendbar. Bei einer Ausbildung des Bürstenkopfes 4 als Interdentalbürstchen wird vorzugsweise für den Draht 7 ein kunststoffbeschichteter Stahl- oder Edelstahldraht verwendet.

Die Längserstreckung des Bürstenkopfes 4 ist wählbar und vorzugsweise an die Länge des Wattepolsters 5 angepaßt. Gleiches gilt vorzugsweise auch für die Quererstreckung des Bürstenkopfes 4.

Die Befestigung des Bürstenkopfes 4 an dem Griffkörper 1 kann durch Schweißen oder Kleben erfolgen. Wird der Griffkörper 1 von einer extrudierten Hülse gebildet, ist der Bürstenkopf 4 vorzugsweise mit einem Befestigungsende 10 in die als Griffkörper 1 ausgebildete Hülse eingeklebt, und zwar vorzugsweise mittels eines härtenden Kunstharzes.

Der Griffkörper 1 ist im allgemeinen ein runder oder elliptischer Stab in Voll- oder Hohlausbildung. Als Material für den Griffkörper 1 wird bevorzugt ein elastischer Kunststoff, insbesondere Polypropylen oder Polystyrol, eingesetzt. Ferner einsetzbar sind hochelastische Kunststoffe, wie Celluloseester und insbesondere Cellulosepropionat oder Celluloseacetopropionat.

Figur 2 zeigt ein zweites Ausführungsbeipiel, das sich von dem vorstehend beschriebenen ersten Ausführungsbeispiel dadurch unterscheidet, daß der Griffkörper 1 an beiden freien Enden 2, 3 einen Bürstenkopf 4 trägt, für den die vorstehenden Ausführungen jeweils entsprechend gelten.

Figur 3 zeigt ein drittes Ausführungsbeispiel, das sich von dem vorstehend beschriebenen ersten Ausführungsbeispiel dadurch unterscheidet, daß als zweiter Pflegekörper an dem zweiten Ende 3 ein Zahnstocher 11 vorgesehen ist. Im übrigen gelten die vorstehenden Ausführungen entsprechend.

Figur 4 zeigt ein viertes Ausführungsbeispiel, das sich von dem vorstehend beschriebenen ersten Ausführungsbeispiel dadurch unterscheidet, daß als zweiter Pflegekörper an dem ersten Ende 2 ein Zahnseidenhalter 12 mit Zahnseide 13 vorgesehen ist und der dargestellte Bürstenkopf 4 eine zylinderförmige Anordnung der Borsten 6 besitzt. Der Zahnseidenhalter 12 ist hier als Spannbügel mit eingespannter Zahnseide 13 ausgebildet. Alternativ kann der Zahnseidenhalter auch als Halter für das Feshalten eines Endes eines losen Zahnseidenfadens ausgebildet sein. Im übrigen gelten die vorstehenden Ausführungen entsprechend, wobei für den Zahnseidenhalter 12 die Kunststoffmaterialien wie für den Griffkörper 1 einsetzbar sind.

## Patentansprüche

1. Pflegestäbchen mit einem länglichen Griffkörper, an dessen beiden freien Enden jeweils ein Pflegekörper angeordnet ist, dadurch gekennzeichnet, daß mindestens ein Pflegekörper (4) gebildet ist von einem Bürstenkopf (6) mit entlang eines Drahtes (7) befestigten Borsten, und ein überstehendes freies Ende (8) des Drahtes (7) von einem tropfenförmigen Polymerüberzug (9) umschlossen ist.

2. Pflegestäbchen nach Anspruch 1, dadurch gekennzeichnet, daß der tropfenförmige Polymerüberzug (9) ein gehärteter Duromertropfen ist.

3. Pflegestäbchen nach Anspruch 2, dadurch gekennzeichnet, daß als Duromer ein UP-Harz, Vinylesterharz oder Epoxidharz eingesetzt ist.

4. Pflegestäbchen nach Anspruch 1, dadurch gekennzeichnet, daß der tropfenförmige Polymerüberzug (9) aus einem Thermoplast besteht.

5. Pflegestäbchen nach einem der Ansprüche 1 bis 4, dadurch gekennzeichnet, daß der Bürstenkopf (4) als Interdentalbürstchen ausgebildet ist.

6. Pflegestäbchen nach einem der Ansprüche 1 bis 5, dadurch gekennzeichnet, daß der Griffkörper (1) eine extrudierte Hülse ist, in die der Bürstenkopf (4) eingeklebt ist.

7. Pflegestäbchen nach einem der Ansprüche 1 bis 6, dadurch gekennzeichnet, daß der Bürstenkopf (4) als Flach- oder Rundbürste ausgebildet ist.

8. Pflegestäbchen nach einem der Ansprüche 1 bis 7, dadurch gekennzeichnet, daß die Borsten (6) des Bürstenkopfes (4) aus einem Natur- oder Kunststoffmaterial bestehen.

9. Pflegestäbchen nach einem der Ansprüche 1 bis 8, dadurch gekennzeichnet, daß der Griffkörper (1) aus einem elastischen Kunststoffmaterial besteht.

10. Pflegestäbchen nach einem der Ansprüche 1 bis 9, dadurch gekennzeichnet, daß als Kunststoffmaterial für den Griffkörper (1) ein Celluloseester vorgesehen ist.

11. Pflegestäbchen nach einem der Ansprüche 1 bis 10, dadurch gekennzeichnet, daß der Pflegekörper an dem anderen Ende (3) von einem zweiten Bürstenkopf (4) oder von einem anderen Pflegekörper (5, 11) gebildet ist.

12. Pflegestäbchen nach Anspruch 11, dadurch gekennzeichnet, daß als anderer Pflegekörper ein Wattepolster (5), ein Zahnstocher (11) oder ein Zahnseidenhalter (12) vorgesehen ist.
